# EUROPEAN PATENT SPECIFICATION

(11) **EP 1 017 802 B1**
(45) Date of publication and mention of the grant of the patent: **08.09.2004**
(21) Application number: 98944896.4
(22) Date of filing: 23.09.1998
(51) Int. Cl.: C12N 15/11, C12N 15/12, C07K 14/46, C07K 19/00, C07K 16/18, C12Q 1/68, G01N 33/68

(54) **A POTENTIAL EFFECTOR FOR THE GRB7 FAMILY OF SIGNALLING PROTEINS**
POTENTIELLER EFFEKTOR DER GRB7-FAMILIE VON SIGNALPROTEINEN
EFFECTEUR POTENTIEL POUR LA FAMILLE Grb7 DES PROTEINES DE SIGNALISATION

(30) Priority: 23.09.1997 AU PO938897
(43) Date of publication of application: 12.07.2000
(73) Proprietor: GARVAN INSTITUTE OF MEDICAL RESEARCH, Darlinghurst, NSW 2010 (AU)
(72) Inventor: DALY, Roger, John, Alexandria, NSW 2015 (AU); SUTHERLAND, Robert, Lyndsay, Lindfield, NSW 2070 (AU)
(74) Representative: Harding, Charles Thomas
(86) International application number: PCT/AU1998/000795
(87) International publication number: WO 1999/015647

(56) References cited:
- WO-A-99/64606
- DATABASE EM_EST [Online] EMBL; Identifier : HS714133; Accession number : R64714, 4 June 1995 (1995-06-04) BOUILLAUD, F.: "EST54a09 WATM1 Homo sapiens cDNA clone 54a09 " XP002154756
- JANES P.W. et al., "Structural Determinants of the Interaction Between the erbB2 Receptor and the Src Homology 2 Domain of Grb7", THE JOURNAL OF BIOLOGICAL CHEMISTRY, Volume 272(13), (1997), pages 8490-8497.
- KEEGEN K. and COOPER J.A., "Use of the Two Hybrid Systems to Detect the Association of the Protein-Tyrosine-Phosphatase, SHPTP2, with Another SH2-Containing Protein, Grb7", ONCOGENE, Volume 12, pages 1537-1544.

### SEQUENCE LISTING

<110> Garvan Institute of Medical Research
<120> A potential effector for the Grb7 family of signalling proteins.
<130> Application number:98944896.4
<140>
   <141>
<160> 2
<170> PatentIn Ver. 2.0
<210> 1
   <211> 3400
   <212> DNA
   <213> Homo sapiens
<400> 1
   attcctcttc ataatgcatg ctcttttggt catgctgaag tagtcaatct ccttttgcga 60
   catggtgcag accccaatgc tcgagataat tggaattata ctcctctcca tgaagctgca 120
   attaaaggaa agattgatgt ttgcattgtg ctgttacagc atggagctga gccaaccatc 180
   cgaaatacag atggaaggac agcattggat ttagcagatc catctgccaa agcagtgctt 240
   actggtgaat ataagaaaga tgaactctta gaaagtgcca ggagtggcaa tgaagaaaaa 300
   atgatggctc tactcacacc attaaatgtc aactgccacg caagtgatgg cagaaagtca 360
   actccattac atttggcagc aggatataac agagtaaaga ttgtacagct gttactgcaa 420
   catggacgtg atgtccatgc taaagataaa ggtgatctgg taccattaca caatgcctgt 480
   tcttatggtc attatgaagt aactgaactt ttggtcaagc atggtggctg tgtaaatgca 540
   atggacttgt ggcaattcac tcctcttcat gaggcagctt ctaagaacag ggttgaagta 600
   tgttctcttc tcttaagtta tggtgcagac ccaacactgc tcaattgtaa gaataaaagt 660
   gctatagact tggctcccac accacagtta aaagaaagat tagcatatga atttaaaggc 720
   cactcgttgc tgcaagctgc acgagaagct gatgttactc gaatcaaaaa acatctctct 780
   ctggaaatgg tgaatttcaa gcatcctcaa acacatgaaa cagcattgca ttgtgctgct 840
   gcatctccat atcccaaaag aaagcaaata tgtgaactgt tgctaagaaa aggagcaaac 900
   atcaatgaaa agactaaaga attcttgact cctctgcacg tggcatctga gaaagctcat 960
   aatgatgttg ttgaagtagt ggtgaaacat gaagcaaagg ttaatgctct ggataatctt 1020
   ggtcagactt ctctacacag agctgcatat tgtggtcatc tacaaacctg ccgcctactc 1080
   ctgagctatg ggtgtgatcc taacattata tcccttcagg gctttactgc tttacagatg 1140
   ggaaatgaaa atgtacagca actcctccaa gagggtatct cattaggtaa ttcagaggca 1200
   gacagacaat tgctggaagc tgcaaaggct ggagatgtcg aaactgtaaa aaaactgtgt 1260
   actgttcaga gtgtcaactg cagagacatt gaagggcgtc agtctacacc acttcatttt 1320
   gcagctgggt ataacagagt gtccgtggtg gaatatctgc tacagcatgg agctgatgtg 1380
   catgctaaag ataaaggagg ccttgtacct ttgcacaatg catgttctta cggacattat 1440
   gaagttgcag aacttcttgt taaacatgga gcagtagtta atgtagctga tttatggaaa 1500
   tttacacctt tacatgaagc agcagcaaaa ggaaaatatg aaatttgcaa acttctgctc 1560
   cagcatggtg cagaccctac aaaaaaaaac agggatggaa atactccttt ggatcttgtt 1620
   aaagatggag atacagatat tcaagatctg cttaggggag atgcagcttt gctagatgct 1680
   gccaagaagg gttgtttagc cagagtgaag aagttgtctt ctcctgataa tgtaaattgc 1740
   cgcgataccc aaggcagaca ttcaacacct ttacatttag cagctggtta taataattta 1800
   gaagttgcag agtatttgtt acaacacgga gctgatgtga atgcccaaga caaaggagga 1860
   cttattcctt tacataatgc agcatcttac gggcatgtag atgtagcagc tctactaata 1920
   aagtataatg catctctcaa tgccacggac aaatgggctt tcacaccttt gcacgaagca 1980
   gcccaaaagg gacgaacaca gctttgtgct ttgttgctag cccatggagc tgacccgact 2040
   cttaaaaatc aggaaggaca aacaccttta gatttagttt cagcagatga tgtcagcgct 2100
   cttctgacag cagccatgcc cccatctgct ctgccctctt gttacaagcc tcaagtgctc 2160
   aatggtgtga gaagcccagg agccactgca gatgctctct cttcaggtcc atctagccca 2220
   tcaagccttt ctgcagccag cagtcttgac aacttatctg ggagtttttc agaactgtct 2280
   tcagtagtta gttcaagtgg aacagagggt gcttccagtt tggagaaaaa ggaggttcca 2340
   ggagtagatt ttagcataac tcaattcgta aggaatcttg gacttgagca cctaatggat 2400
   atatttgaga gagaacagat cactttggat gtattagttg agatggggca caaggagctg 2460
   aaggagattg gaatcaatgc ttatggacat aggcacaaac taattaaagg agtcgagaga 2520
   cttatctccg gacaacaagg tcttaaccca tatttaactt tgaacacctc tggtagtgga 2580
   acaattctta tagatctgtc tcctgatgat aaagagtttc agtctgtgga ggaagagatg 2640
   caaagtacag ttcgagagca cagagatgga ggtcatgcag gtggaatctt caacagatac 2700
   aatattctca agattcagaa ggtttgtaac aagaaactat gggaaagata cactcaccgg 2760
   agaaaagaag tttctgaaga aaaccacaac catgccaatg aacgaatgct atttcatggg 2820
   tctccttttg tgaatgcaat tatccacaaa ggctttgatg aaaggcatgc gtacataggt 2880
   ggtatgtttg gagctggcat ttattttgct gaaaactctt ccaaaagcaa tcaatatgta 2940
   tatggaattg gaggaggtac tgggtgtcca gttcacaaag acagatcttg ttacatttgc 3000
   cacaggcagc tgctcttttg ccgggtaacc ttgggaaagt ctttcctgca gttcagtgca 3060
   atgaaaatgg cacattctcc tccaggtcat cactcagtca ctggtaggcc cagtgtaaat 3120
   ggcctagcat tagctgaata tgttatttac agaggagaac aggcttatcc tgagtattta 3180
   attacttacc agattatgag gcctgaaggt atggtcgatg gataaatagt tattttaaga 3240
   aactaattcc actgaaccta aaatcatcaa agcagcagtg gcctctacgt tttactcctt 3300
   tgctgaaaaa aaatcatctt gcccacaggc ctgtggcaaa aggataaaaa tgtgaacgaa 3360
   gtttaacatt ctgacttgat aaagctttaa taatgtacag                       3400
<210> 2
   <211> 1074
   <212> PRT
   <213> Homo sapiens
<400> 2
   Ile Pro Leu His Asn Ala Cys Ser Phe Gly His Ala Glu Val Val Asn
    1               5                   10                  15
   Leu Leu Leu Arg His Gly Ala Asp Pro Asn Ala Arg Asp Asn Trp Asn
               20                  25                  30
   Tyr Thr Pro Leu His Glu Ala Ala Ile Lys Gly Lys Ile Asp Val Cys
           35                  40                  45
   Ile Val Leu Leu Gln His Gly Ala Glu Pro Thr Ile Arg Asn Thr Asp
           50                  55                  60
   Gly Arg Thr Ala Leu Asp Leu Ala Asp Pro Ser Ala Lys Ala Val Leu
    65                  70                  75                  80
   Thr Gly Glu Tyr Lys Lys Asp Glu Leu Leu Glu Ser Ala Arg Ser Gly
                    85                  90                  95
   Asn Glu Glu Lys Met Met Ala Leu Leu Thr Pro Leu Asn Val Asn Cys
               100                 105                 110
   His Ala Ser Asp Gly Arg Lys Ser Thr Pro Leu His Leu Ala Ala Gly
           115                 120                 125
   Tyr Asn Arg Val Lys Ile Val Gln Leu Leu Leu Gln His Gly Arg Asp
       130                 135                 140
   Val His Ala Lys Asp Lys Gly Asp Leu Val Pro Leu His Asn Ala Cys
   145                 150                 155                 160
   Ser Tyr Gly His Tyr Glu Val Thr Glu Leu Leu Val Lys His Gly Gly
                   165                 170                 175
   Cys Val Asn Ala Met Asp Leu Trp Gln Phe Thr Pro Leu His Glu Ala
               180                 185                 190
   Ala Ser Lys Asn Arg Val Glu Val Cys Ser Leu Leu Leu Ser Tyr Gly
           195                 200                 205
   Ala Asp Pro Thr Leu Leu Asn Cys Lys Asn Lys Ser Ala Ile Asp Leu
       210                 215                 220
   Ala Pro Thr Pro Gln Leu Lys Glu Arg Leu Ala Tyr Glu Phe Lys Gly
   225                 230                 235                 240
   His Ser Leu Leu Gln Ala Ala Arg Glu Ala Asp Val Thr Arg Ile Lys
                   245                 250                 255
   Lys His Leu Ser Leu Glu Met Val Asn Phe Lys His Pro Gln Thr His
               260                 265                 270
   Glu Thr Ala Leu His Cys Ala Ala Ala Ser Pro Tyr Pro Lys Arg Lys
           275                 280                 285
   Gln Ile Cys Glu Leu Leu Leu Arg Lys Gly Ala Asn Ile Asn Glu Lys
       290                 295                 300
   Thr Lys Glu Phe Leu Thr Pro Leu His Val Ala Ser Glu Lys Ala His
   305 310 315 320
   Asn Asp Val Val Glu Val Val Val Lys His Glu Ala Lys Val Asn Ala
   325 330 335
   Leu Asp Asn Leu Gly Gln Thr Ser Leu His Arg Ala Ala Tyr Cys Gly
   340 345 350
   His Leu Gln Thr Cys Arg Leu Leu Leu Ser Tyr Gly Cys Asp Pro Asn
   355 360 365
   Ile Ile Ser Leu Gln Gly Phe Thr Ala Leu Gln Met Gly Asn Glu Asn
   370 375 380
   Val Gln Gln Leu Leu Gln Glu Gly Ile Ser Leu Gly Asn Ser Glu Ala
   385 390 395 400
   Asp Arg Gln Leu Leu Glu Ala Ala Lys Ala Gly Asp Val Glu Thr Val
   405 410 415
   Lys Lys Leu Cys Thr Val Gln Ser Val Asn Cys Arg Asp Ile Glu Gly
   420 425 430
   Arg Gln Ser Thr Pro Leu His Phe Ala Ala Gly Tyr Asn Arg Val Ser
   435 440 445
   Val Val Glu Tyr Leu Leu Gln His Gly Ala Asp Val His Ala Lys Asp
   450 455 460
   Lys Gly Gly Leu Val Pro Leu His Asn Ala Cys Ser Tyr Gly His Tyr
   465 470 475 480
   Glu Val Ala Glu Leu Leu Val Lys His Gly Ala Val Val Asn Val Ala
   485 490 495
   Asp Leu Trp Lys Phe Thr Pro Leu His Glu Ala Ala Ala Lys Gly Lys
   500 505 510
   Tyr Glu Ile Cys Lys Leu Leu Leu Gln His Gly Ala Asp Pro Thr Lys
   515 520 525
   Lys Asn Arg Asp Gly Asn Thr Pro Leu Asp Leu Val Lys Asp Gly Asp
   530 535 540
   Thr Asp Ile Gln Asp Leu Leu Arg Gly Asp Ala Ala Leu Leu Asp Ala
   545 550 555 560
   Ala Lys Lys Gly Cys Leu Ala Arg Val Lys Lys Leu Ser Ser Pro Asp
                   565                 570                 575
   Asn Val Asn Cys Arg Asp Thr Gln Gly Arg His Ser Thr Pro Leu His
               580                 585                 590
   Leu Ala Ala Gly Tyr Asn Asn Leu Glu Val Ala Glu Tyr Leu Leu Gln
           595                 600                 605
   His Gly Ala Asp Val Asn Ala Gln Asp Lys Gly Gly Leu Ile Pro Leu
       610                 615                 620
   His Asn Ala Ala Ser Tyr Gly His Val Asp Val Ala Ala Leu Leu Ile
   625                 630                 635                 640
   Lys Tyr Asn Ala Ser Leu Asn Ala Thr Asp Lys Trp Ala Phe Thr Pro
                   645                 650                 655
   Leu His Glu Ala Ala Gln Lys Gly Arg Thr Gln Leu Cys Ala Leu Leu
               660                 665                 670
   Leu Ala His Gly Ala Asp Pro Thr Leu Lys Asn Gln Glu Gly Gln Thr
           675                 680                 685
   Pro Leu Asp Leu Val Ser Ala Asp Asp Val Ser Ala Leu Leu Thr Ala
       690                 695                 700
   Ala Met Pro Pro Ser Ala Leu Pro Ser Cys Tyr Lys Pro Gln Val Leu
   705                 710                 715                 720
   Asn Gly Val Arg Ser Pro Gly Ala Thr Ala Asp Ala Leu Ser Ser Gly
                   725                 730                 735
   Pro Ser Ser Pro Ser Ser Leu Ser Ala Ala Ser Ser Leu Asp Asn Leu
               740                 745                 750
   Ser Gly Ser Phe Ser Glu Leu Ser Ser Val Val Ser Ser Ser Gly Thr
           755                 760                 765
   Glu Gly Ala Ser Ser Leu Glu Lys Lys Glu Val Pro Gly Val Asp Phe
       770                 775                 780
   Ser Ile Thr Gln Phe Val Arg Asn Leu Gly Leu Glu His Leu Met Asp
   785                 790                 795                 800
   Ile Phe Glu Arg Glu Gln Ile Thr Leu Asp Val Leu Val Glu Met Gly
                   805                 810                 815
   His Lys Glu Leu Lys Glu Ile Gly Ile Asn Ala Tyr Gly His Arg His
               820                 825                 830
   Lys Leu Ile Lys Gly Val Glu Arg Leu Ile Ser Gly Gln Gln Gly Leu
           835                 840                 845
   Asn Pro Tyr Leu Thr Leu Asn Thr Ser Gly Ser Gly Thr Ile Leu Ile
       850                 855                 860
   Asp Leu Ser Pro Asp Asp Lys Glu Phe Gln Ser Val Glu Glu Glu Met
   865                 870                 875                 880
   Gln Ser Thr Val Arg Glu His Arg Asp Gly Gly His Ala Gly Gly Ile
                   885                 890                 895
   Phe Asn Arg Tyr Asn Ile Leu Lys Ile Gln Lys Val Cys Asn Lys Lys
               900                 905                 910
   Leu Trp Glu Arg Tyr Thr His Arg Arg Lys Glu Val Ser Glu Glu Asn
           915                 920                 925
   His Asn His Ala Asn Glu Arg Met Leu Phe His Gly Ser Pro Phe Val
       930                 935                 940
   Asn Ala Ile Ile His Lys Gly Phe Asp Glu Arg His Ala Tyr Ile Gly
   945                 950                 955                 960
   Gly Met Phe Gly Ala Gly Ile Tyr Phe Ala Glu Asn Ser Ser Lys Ser
                   965                 970                 975
   Asn Gln Tyr Val Tyr Gly Ile Gly Gly Gly Thr Gly Cys Pro Val His
               980                 985                 990
   Lys Asp Arg Ser Cys Tyr Ile Cys His Arg Gln Leu Leu Phe Cys Arg
           995                 1000                1005
   Val Thr Leu Gly Lys Ser Phe Leu Gln Phe Ser Ala Met Lys Met Ala
       1010                1015                1020
   His Ser Pro Pro Gly His His Ser Val Thr Gly Arg Pro Ser Val Asn
   1025                1030                1035                1040
   Gly Leu Ala Leu Ala Glu Tyr Val Ile Tyr Arg Gly Glu Gln Ala Tyr
                   1045                1050                1055
   Pro Glu Tyr Leu Ile Thr Tyr Gln Ile Met Arg Pro Glu Gly Met Val
               1060                1065                1070
   Asp Gly

## Description

### Field of the Invention:

The present invention relates to a novel polynucleotide molecule encoding a candidate effector protein for the Grb7 family of signalling proteins. Detection of the encoded protein in a tissue sample should provide a useful tumour marker and/or prognostic indicator. Furthermore, antagonism of the interaction between Grb7 family members and the encoded protein should provide a novel treatment strategy for human diseases exhibiting aberrant receptor tyrosine kinase (RTK) signalling (e.g. cancer).

### Background of the Invention

RTKs play a major role in the regulation of cellular growth, differentiation, motility and metabolism by converting an extracellular signal in the form of the binding of a specific hormone or growth factor to the activation of specific signalling pathways and hence modes of intracellular communication (Schlessinger and Ullrich, *Neuron* 9. 383-391. 1992). Activation of RTKs results in both autophosphorylation of the receptor and the phosphorylation of downstream targets on tyrosine residues. It has become evident over the last decade that key elements in receptor-substrate and other protein-protein interactions in RTK signalling are src homology (SH)2 domains. SH2 domains are conserved modules of approximately 100 amino acids found in a wide variety of signalling molecules which bind to short tyrosine-phosphorylated peptide sequences. The specificity of interaction is determined both by the nature of the amino acids flanking the phosphotyrosine residue in the target peptide and residues in the SH2 domain which interact with these sites (Pawson, *Nature* 373. 573-580. 1995).

SH2-domain containing proteins can be divided into two classes: those which possess a catalytic function (e.g. the cytoplasmic tyrosine kinase c-src and the tyrosine phosphatase SH-PTP2) and those which consist entirely of non-catalytic protein domains (eg Grb2). the adaptor sub-class. The function of the latter class is to link separate catalytic subunits to a tyrosine-phosphorylated receptor or signalling intermediate, and other non-catalytic protein modules are often involved in these interactions. For example, SH3 and WW domains (conserved regions of approximately 50 and 40 amino acids, respectively) bind proline-rich peptide ligands, and pleckstrin homology domains (approximately 100 amino acids) interact with both specific phospholipid and protein targets (Pawson, 1995 *supra*).

The Grb7 family represents a family of SH2 domain-containing adaptors which currently contains three members: Grb7, 10 and 14 (Margolis *et al*, *Proc. Natl. Acad*. *Sci. USA* 89, 8894-8898, 1992; Stein *et al*, *EMBO J* 13, 1331-1340, 1994; Ooi *et al*, *Oncogene* 10, 1621-1630, 1995; Daly *et al*, *J. Biol. Chem.* 271, 12502-12510, 1996). These proteins share a common overall architecture, consisting of an N-terminal region containing a highly conserved proline-rich decapeptide motif, a central region harbouring a PH domain and a C-terminal SH2 domain. The central region of approximately 300 amino acids bears significant homology to the *C*. *elegans* protein mig 10, which is required for long range neuronal migration in embryos, otherwise the Grb7 family and mig10 are structurally distinct. However, they exhibit differences in both SH2 selectivity towards RTKs (Janes *et al*, *J Biol*. *Chem.* 272, 8490-8497, 1997) and tissue distribution. The family has therefore evolved to link particular receptors to downstream effectors in a tissue-specific manner. Interestingly, the genes encoding this family appear to have co-segregated with *ERBB* family genes during evolution. Thus *GRB7, 10* and *14* are linked to *ERBB2, ERBB1* (epidermal growth factor receptor) and *ERBB4,* respectively (Stein *et al* 1994 *supra*; Ooi *et al*, 1995 *supra*; Baker *et al*, *Genomics* 36, 218-220, 1996). The juxtaposition of *GRB7* and *ERBB2* leads to common co-amplification in human breast cancers, and since the two gene products are functionally linked, likely up-regulation of an undefined erbB2 signalling pathway. Furthermore, *GRB14* also exhibits differential expression in human breast cancers (Daly *et al*, 1996 *supra*). These two proteins may therefore modulate RTK signalling in this disease.

In order to identify proteins which bind to this family and therefore identify candidate effectors, we performed a genetic screen using the yeast two hybrid system and Grb14 "bait". This application describes the cloning and characterization of a novel interacting protein, currently designated 2.2412, the amino acid sequence of which is set forth in SEQ ID NO: 2. The nucleotide sequence encoding this interacting protein is set forth in SEQ ID NO: 1.

Bouillaud (EMBL Database abstract with accession number R64714, June 4, 1995) disclose the nucleotide sequence of an Expressed Sequence Tag (EST) from adipose tissue that consists of 659 nucleotides having high homology to nucleotides 935-1593 of SEQ ID NO: 1.

A similar approach had already been taken by Keegan et al. (ONCOGENE 12 (1996),1537-44) which disclose the use of a yeast two-hybrid system to detect proteins interacting with SHPTP2. This screen identifies Grb7 as such an interacting protein. In the reverse experiment, using Grb7 as a bait, the authors show that SHPTP2 associates with Grb7.

### Disclosure of the Invention:

Thus, in a first aspect, the present invention provides an isolated polynucleotide molecule encoding a candidate effector protein for the Grb7 family of signalling proteins, wherein the polynucleotide molecule comprises a nucleotide sequence having at least 75% sequence identity to that shown as SEQ ID NO: 1. As exemplified herein, the encoded candidate effector protein for the Grb7 family of signalling proteins binds to both Grb7 and Grb14 proteins.
Preferably, the polynucleotide molecule comprises a nucleotide sequence having at least 85%, more preferably at least 95%, sequence identity to that shown as SEQ ID NO: 1. Most preferably, the polynucleotide molecule comprises a nucleotide sequence encoding a polypeptide comprising the amino acid sequence set forth in SEQ ID NO: 2.

In a preferred embodiment of the invention of the first aspect, the polynucleotide molecule comprises the nucleotide sequence set forth in SEQ ID NO: 1.

The polynucleotide molecule may be a dominant negative mutant which encodes a gene product causing an altered phenotype by, for example, reducing or eliminating the activity of endogenous effector proteins of the Grb7 family of signalling proteins.

The polynucleotide molecule may be incorporated into plasmids or expression vectors (including viral vectors), which may then be introduced into suitable host cells such as bacterial, yeast, insect and mammalian host cells. Such host cells may be used to express the protein encoded by the polynucleotide molecule.

Accordingly, in a second aspect, the present invention provides a host cell transformed with the polynucleotide molecule of the first aspect.

In a third aspect, the present invention provides a method of producing a protein, comprising culturing the host cell of the second aspect under conditions suitable for the expression of the polynucleotide molecule and optionally recovering the protein.

Preferably, the host cell is mammalian or of insect origin. Where the cell is mammalian, it is presently preferred that it be a Chinese hamster ovary (CHO) cell or human embryonic kidney (HEK) 293 cell. Where the host cell is of insect origin, it is presently preferred that it be an insect Sf9 cell.

In a fourth aspect, the present invention provides a purified protein encoded by the polynucleotide molecule of the first aspect.

In a preferred embodiment of this aspect, the purified protein comprises an amino acid sequence shown as SEQ ID NO: 2.

In a fifth aspect, the present invention provides a fusion protein comprising a candidate effector protein for the Grb7 family of signalling proteins wherein said candidate effector protein comprises an amino acid sequence encoded by the polynucleotide molecule according to the first aspect of the present invention.

In a preferred embodiment of this aspect, the fusion protein comprises the amino acid sequence shown as SEQ ID No. 2.

Fusion proteins according to the fifth aspect may include an N-terminal fragment of a protein such as beta-galactosidase to assist in the expression and selection of host cells expressing candidate effector protein, or may include a functional fragment of any other suitable protein to confer additional activity(ies).

In a sixth aspect, the present invention provides an antibody or fragment thereof which specifically binds to the protein of the fourth aspect.

The antibody may be monoclonal or polyclonal, however, it is presently preferred that the antibody is a monoclonal antibody. Suitable antibody fragments include Fab, F(ab')2 and scFv.

Suitable oligonucleotide probes which may be used to selectively hybridise to the polynucleotide molecule of the first aspect under high stringency conditions (Sambrook et al., Molecular Cloning: a Laboratory Manual. Second Edition. Cold Spring Harbor Laboratory Press) comprise a nucleotide sequence of at least 12 nucleotides. Preferably, the oligonucleotide probe is labelled. More preferably, the oligonucleotide probe comprises a nucleotide sequence of at least 18 nucleotides.

In a seventh aspect, the present invention provides a method of detecting in a sample the presence of an effector protein for the Grb7 family of proteins, the method comprising reacting the sample with an antibody or fragment thereof according to the sixth aspect, and detecting the binding of the antibody or fragment thereof.

The method of the seventh aspect may be conducted using any immunoassays well known in the art (e.g. ELISA). The sample may be, for example, a cell lysate or homogenate prepared from a tissue biopsy.

In an eighth aspect, the present invention provides a method of detecting in a sample the presence of mRNA encoding an effector protein for the Grb7 family of proteins, the method comprising reacting the sample with an oligonucleotide probe of the seventh aspect, and detecting the binding of the probe.

The method of the eighth aspect may be conducted using any hybridisation assays well known in the art (e.g. Northern blot). The sample may be a poly(A) RNA preparation or homogenate prepared from a tissue biopsy.

Grb7 family proteins exhibit differential expression in certain human cancers (particularly breast and prostate cancer) and may therefore be involved in tumour progression. Detection of the protein encoded by the cDNA 2.2412 in a sample should provide a useful tumour marker and/or prognostic indicator for these cancers. Furthermore, the interaction of Grb7 family members with 2.2412 may provide a novel target for therapeutic intervention.

It is to be understood that methods of detecting suitable agonists and methods of therapy utilising detected agonists also form part of the present invention.

The present invention encompass minor variations in the nucleotide sequence of SEQ ID No. 1 which due to degeneracy in the DNA code do not result in a change in the encoded protein. Further, other minor variations in the sequence which may be required to enhance expression in a particular system but in which the variations do not result in a decrease in biological activity of the encoded protein are also encompassed.

The present invention encompass minor variations in the sequence of SEQ ID No. 2 which do not result in a decrease in biological activity of the protein. These variations may include conservative amino acid substitutions. The substitutions envisaged are:-
G, A, V, I, L, M: D, E; N, Q; S, T; K, R, H: F, Y, W, H: and
P. Nα-alkalamino acids.

The terms "comprise", "comprises" and "comprising" as used throughout the specification are intended to refer to the inclusion of a stated step, component or feature of group of steps, components of features with or without the inclusion of a further step. component or feature or group of steps, components or features.

The invention will hereinafter be described with reference to the accompanying figure and the following, non-limiting example.

### Brief description of the accompanying figure:

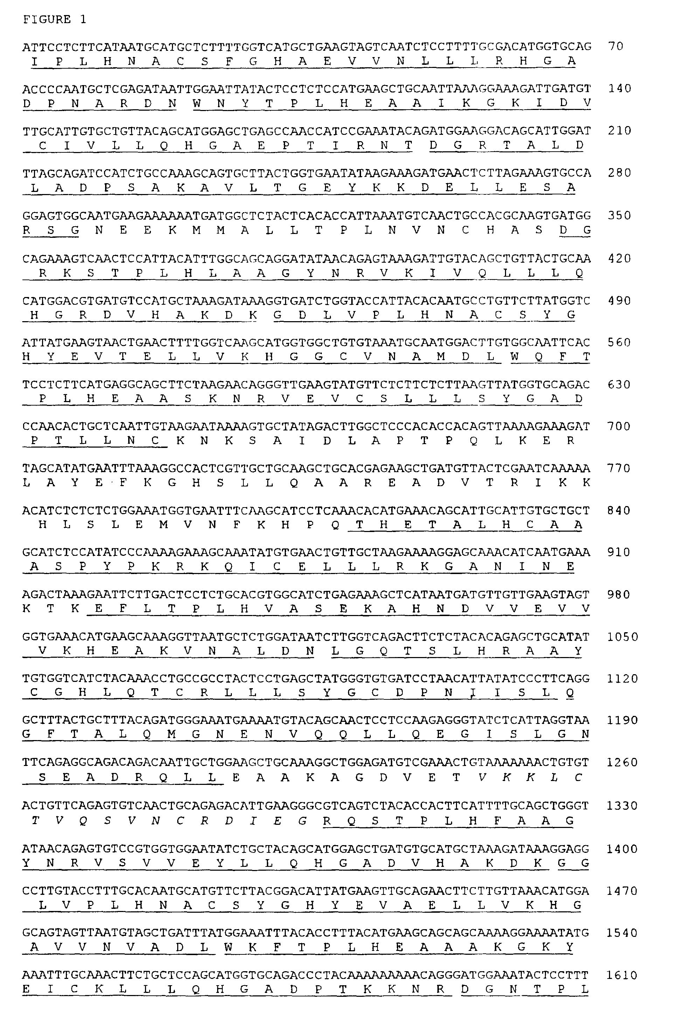

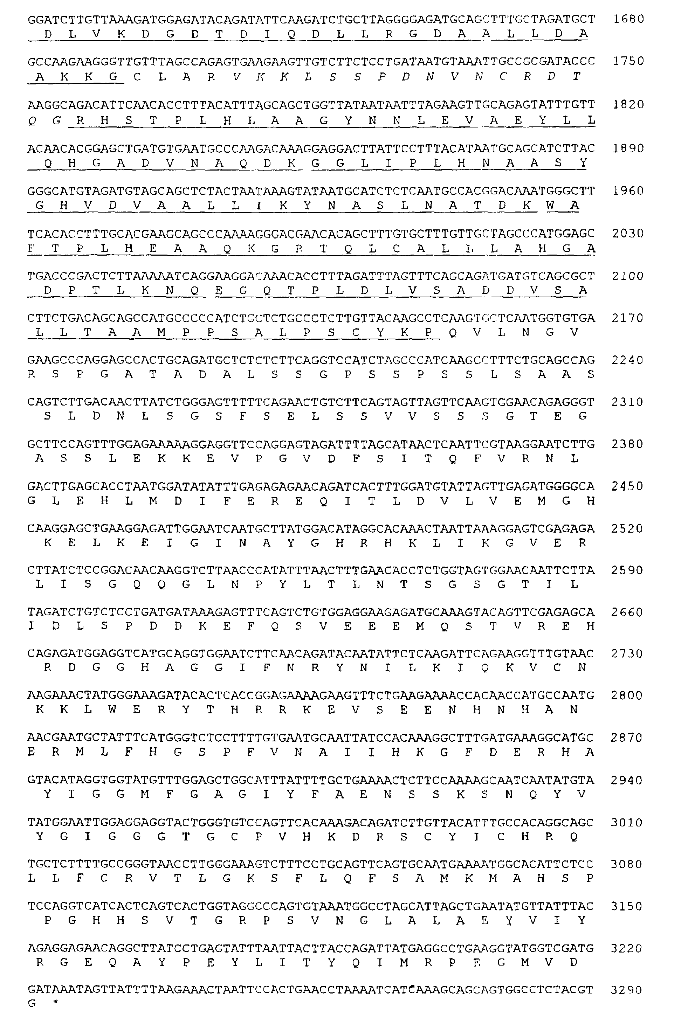

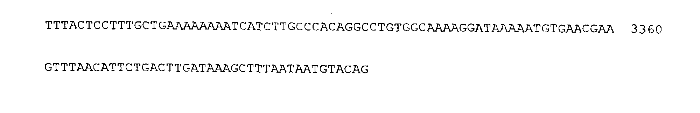

**Figure 1** provides the nucleotide and amino acid (single letter code) sequence of 2.2412. Numbers refer to distances in base pairs. Ankyrin-type repeat sequences are underlined. An additional repeat sequence is indicated by italics. The stop codon is represented by an asterisk. The original cDNA clone 2.2412 isolated by the two hybrid screen spans nucleotides 694-2664 of this sequence.

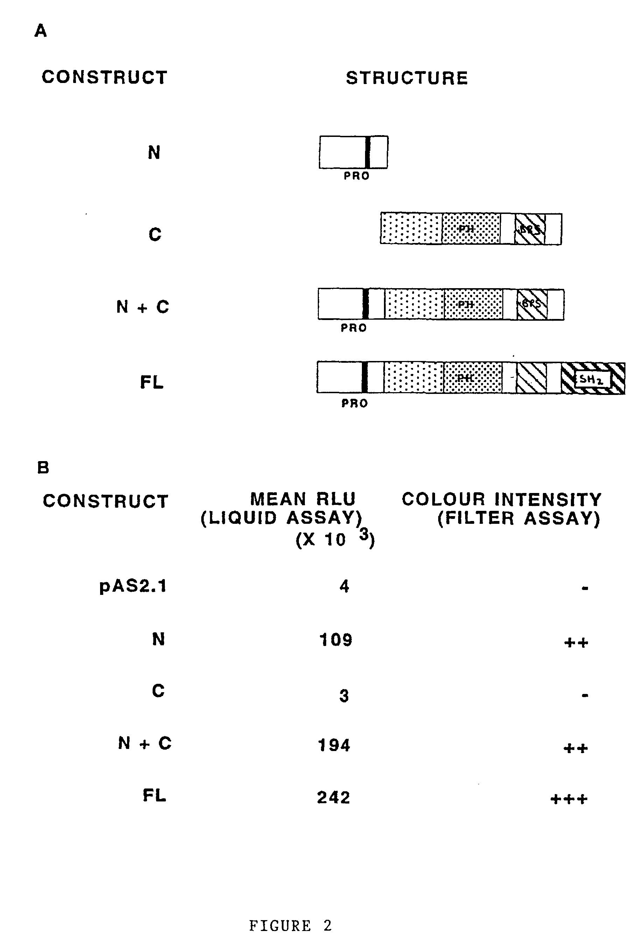

**Figure 2** provides a map of the 2.2412-binding region on Grb14. A. Structure of the deletion constructs used in the analysis Gal4 DNA-BD fusion constructs encoding full length Grb14 (FL), the N-terminal (N). central region (C) and N-terminal + central region (N + C) were generated in the vector pAS2.1. B. Results of β-galactosidase activity assays following transformation of the above plasmids into yeast strain Y190 together with the original 2.2412 cDNA clone in pACT-2.

### Example: CLONING AND CHARACTERISATION OF 2.2412

### Yeast two hybrid screen

The yeast two hybrid system exploits protein-protein interactions to reconstitute a functional transcriptional activator which can then be detected using a gene reporter system (Fields and Sternglanz. *TIG*. 10. 286-292. 1994). The technique takes advantage of the properties of the Gal4 protein of the yeast *S. cerevisiae*. The Gal4 DNA binding domain (DNA-BD) or activation domain (AD) alone are incapable of inducing transcription. However, an interaction between two proteins synthesized as DNA-BD- and AD-fusions, respectively, brings the Gal4 domains into close proximity and results in transcriptional activation of two reporter genes (*HIS3* and *LacZ)* which can be monitored by growth on selective medium and biochemical assays.

A plasmid construct encoding a Gal4 DNA-BD-Grb14 fusion was generated as follows. The plasmid *GRB14*/pRcCMV_{F} containing full length *GRB14* cDNA (Daly *et al*. 1996) was restricted with HindIII and Klenow treated to create blunt ends. and then digested with *BclI* to release three fragments of approximately 1.1, 4.2 and 1.7 kb. The 1.7 kb fragment was isolated and cloned into the *NdeI* (Klenow treated) and *Bam*HI sites of the yeast expression vector pAS2.1 (Clontech) to generate *GRB14*/pAS2.1 containing an in-frame fusion of full length Grb14 with the GAL4 DNA-BD. This construct was introduced by electroporation into the yeast strain CG1945 (*MATa, ura3-52, his3-200, ade2-101, lys2-801,trp1-901, leu2-3, 112, gal4-542, gal80-538, cyh*^{*r*}*2, LYS2::GAL1*_{*UAS*}*-GAL1*_{*TATA*}*-HIS3, URA3::GAL4*_{*17mers(x3)*}*-CYC1*_{*TATA*}*-lacZ*) selecting for tryptophan prototrophy. The expression of the fusion protein was verified by Western blot analysis with antibodies directed against the Flag epitope and the Gal4 DNA-BD. The recipient strain was then grown to mid-log phase and a human liver cDNA library in the vector pACT2 (Clontech) introduced using the LiAc procedure (Schiestl and Gietz, *Curr*. *Genet.* 16. 339-346. 1989). Transformants were then selected for tryptophan, leucine and histidine prototrophy in the presence of 5mM 3-aminotriazole.

From a screen of 1x10⁶ clones, 39 colonies were initially selected on synthetic complete (SC)-leu-his-trp +3AT medium and were then tested for β-galactosidase activity. 12 clones scored positive in the latter assay and were subjected to cycloheximide (CHX) curing to remove the bait plasmid by streaking out on SC-leu media containing 10ug/ml CHX (pAS2-1 contains the *CYH2* gene which restores CHX sensitivity to CG1945 cells). This enabled confirmation of the bait dependency of *LacZ* activation and subsequent isolation of the pACT2 plasmids encoding interacting proteins by standard methodology (Philippsen et *al*, *Methods in Enzymology* 194, 170-177). Back transformations were then performed in which these pACT2 plasmids were introduced into CG1945 strains containing the bait plasmid (*GRB14*/pAS2-1) or constructs encoding non-related Gal4 DNA-BD fusions in order to confirm the specificity of the interactions.

The DNA sequences of the cDNA inserts were then obtained by cycle sequencing (f-mol kit. Promega) using pACT2-specific and/or clone-specific primers. Based on their nucleotide sequences the 12 interacting clones were classified into 6 independent groups (see Table I).

**TABLE I:**

| **Characterization of cDNA clones isolated by the yeast two hybrid screen.** | | | | |
|---|---|---|---|---|
| **Class** | **No. of clones** | **Identity** | **Mean RLU (Liquid assay)** | **Colour intensity (Filter assay)** |
| 1 | 6 | Nedd4 | 2.86x10⁶ | ++++ |
| 2 | 2 | Htk | 1.86x10⁵ | ++ |
| 3 | 1 | 2.2412 | 5.18x10⁶ | ++++ |
| 4 | 1 | Proteosome | 3.88x10² | +/- |
| 5 | 1 | Somatostatin | 1.45x10³ | +/- |
| | | receptor | | |
| 6 | 1 | L-arginine:glycine amidinotransferase | 8.61x10² | +/- |

The 12 clones exhibiting activation of both the *HIS3* and *lacZ* reporter genes were divided into 6 groups by sequence analysis of their cDNA inserts. Results of β-galactosidase activity assays performed using two methodologies are shown. The liquid culture-derived method (Galacto-Light, TROPIX) is more quantitative: results are given in mean relative light units (RLU) and are normalized for the protein content of the samples. Blue/white screening of the cDNA clones was also performed using a colony lift filter assay (Clontech). The intensity of blue colour development over approximately 2h is scored from +/- (very weak) to ++++ (strong).

Six clones were partial cDNAs corresponding to Nedd4, a multidomain protein containing a calcium-dependent phospholipid binding (CaLB) domain, four WW domains and a C-terminal region homologous to the E6-AP carboxyl-terminus (Kumar et *al*, *Biochem. Biophys. Res. Commun*. 185, 1155-1161, 1992: Sudol *et al J. Biol. Chem*. 270, 14733-14741. 1995; Huibregtse *et al Proc. Natl. Acad. Sci. USA* 92, 2563-2567, 1995). The latter is likely to confer E3 ubiquitin-protein ligase activity on Nedd4. The pACT2 clones isolated encoded the CaLB domain together with the first 22 amino acids of the first WW domain.

Two clones encoded the intracellular region and part of the extracellular domain of Htk. which is a RTK of the Eph family (Bennett *et al J. Biol. Chem*. 269, 14211-14218. 1994). The recruitment of Grb14 by Htk is of interest for two reasons. First. the expression profile of both Htk and the murine homologue myk-1 are indicative of a potential role in mammary gland development and neoplasia (Andres *et al Oncogene* 9, 1461-1467. 1994: Berclaz *et al Biochem*. *Biophys*. *Res. Comm*. 226. 869-875. 1996). Second, Eph family members may be involved in the regulation of cell migration (Tessier-Lavigne. *Cell* 82. 345-348. 1995), which is intriguing given the homology of the Grb7 family to the *C*. *elegans* protein mig10 (Stein *et al*. 1994 *supra*).

A novel cDNA of 1971 bp. designated 2.2412. was also isolated. This clone encoded a polypeptide of 657 amino acids in frame with the Gal4 DNA-BD. The cDNA did not contain a stop codon, and this, together with the Northern analysis described below, indicated that it was incomplete. This DNA fragment was therefore used as a probe to screen a human placental cDNA library (5' STRETCH PLUS. Clontech. in λgt10). This resulted in the isolation of two clones. designated clone 8 and clone 12. Clone 8 was approximately 2 kb and overlapped the original 2.2412 clone by 900 bp at the 3' end. This clone provided the carboxy-terminal end of the 2.2412 protein sequence (Figure 1). Clone 12 was approximately 3.5 kb and to date has provided an additional 692 bp of sequence information in the 5' direction. The nucleotide and protein sequence for 2.2412 provided by these overlapping clones is shown in Figure 1. Since a 5' initiation codon has yet to be identified the coding sequence still appears to be incomplete.

### Further characterization of 2.2412

Database searches using the 2.2412 cDNA sequence revealed significant homology with a large number of proteins containing ankyrin-like repeats. These sequences were first identified as homologous regions between certain cell cycle regulatory proteins and the Drosophila protein Notch (Breeden and Nasmyth. *Nature* 329, 651-654. 1987) but subsequently they have been identified in a wide variety of other proteins where they are thought to function in protein-protein interactions (Bork, *Proteins* 17. 363-374. 1993). Subsequent analysis of the protein sequence identified 18 consecutive ankyrin repeats and an additional repetitive element (Figure 1). The ankyrin repeat region is followed by a stretch of approximately 40 amino acids rich in serine residues. The remaining C-terminal region has a relatively high content of charged amino acids.

### Northern analysis of 2.2412 mRNA expression

Northern blot analysis of multiple tissue northerns (Clontech) was performed using the original 2.2412 cDNA as a probe. This resulted in the detection of a single mRNA transcript of approximately 7 kb in all tissues examined with the exception of the kidney. Expression was particularly high in skeletal muscle and placenta. The size of this transcript compared to that of the 2.2412 clone indicates that the latter represents only a partial cDNA.

### Genomic localization of the 2.2412 gene

Fluorescence *in situ* hybridization of the original 2.2412 cDNA to normal metaphases (Baker *et al*, 1996 *supra*) and reference to the FRA10A fragile site at 10q23.32 localized the gene to between chromosome 10q23.2 and proximal 10q23.32. Interestingly, deletions in the 10q22-25 region of chromosome 10 have been detected in a variety of human cancers including breast, prostate, renal, small cell lung and endometrial carcinomas, glioblastoma multiforme, melanoma and meningiomas, suggesting the presence of one or more tumour suppressive loci in this region (Li *et al*, *Science* 275, 1943-1947. 1997: Steck et *al*, *Nature Genetics* 15, 356-362. 1997, and references therein). Two candidate tumour suppressor genes have been identified in this region (MMAC1/PTEN and MXI1. Li *et al* 1997 *supra*: Steck *et al* 1997 *supra*; Albarosa *et al*, *Hum. Genet*. 95, 709-711, 1995).

### Analysis of the interaction between 2.2412 and Grb7 family members

cDNAs encoding the full length and N- and C-terminal regions of the original 2.2412 cDNA clone (nucleotides 694-2664, 694-1614 and 1615-2664 of the sequence shown in Figure 1, respectively) were cloned into the vector pGEX4T2 (Pharmacia). The full length construct was generated by subcloning from the pACT2 clone as a NdeI fragment, whereas the shorter constructs were synthesized by directional cloning of PCR products. The corresponding GST-fusion proteins were purified from IPTG-induced bacterial cultures using glutathione-agarose beads (Smith and Johnson, *Gene* 67, 31-40, 1988). These immobilized fusion proteins were then incubated with lysates from cells expressing Flag epitope-tagged Grb14 (Daly *et al*. 1996 *supra*) or human breast cancer cells expressing high levels of Grb7 (SK-BR-3: Stein *et al*. 1994) as described previously (Daly *et al*. 1996). Following washing, bound proteins were detected by Western blot analysis. The results indicated that 2.2412 bound specifically to both Grb14 and Grb7 *in vitro*, and that the N-terminal fusion protein bound more strongly than that derived from the C-terminus. These data, obtained using a different methodology for detecting protein-protein interactions to the yeast two hybrid system, confirm that 2.2412 interacts with Grb14. Furthermore, 2.2412 also binds Grb7. Consequently 2.2412 appears to represent a general effector for the Grb7 family.

### Mapping of the 2.2412 binding region on Grb14

In order to identify the region of Grb14 that interacts with 2.2412. a series of Grb14 deletion mutants were generated by cloning PCR fragments synthesized using the appropriate flanking primers into the vector pAS2.1. These fragments spanned the following regions: N-terminus ("N". amino acids 1-110), the central region ("C") encompassing the mig10 homology and the "between PH and SH2" (BPS) domain (amino acids 110-437) and the N-terminal and central regions ("N + C", amino acids 1-437). These plasmids were individually transformed into the yeast strain Y190 (*MATa, ura3-52, his3-200, ade2-101, lys2-801, trp1-901, leu2-3, 112, gal4Δ, gal80Δ, cyh*^{*r*}*2, LYS2::GAL1* _{*UAS*}*-HIS3*_{*TATA*}*-HIS3, URA3::GAL1* _{*UAS*}*-GAL1*_{*TATA*}*-lacZ*) and expression of the appropriately sized Gal4 DNA-BD fusion proteins confirmed by Western blotting. Following transformation of the resulting yeast strains with the original 2.2412 cDNA clone in pACT-2, the strength of the interaction was determined by either liquid- or filter-based β-galactosidase assays. The results are presented in Figure 2, and demonstrate that the N-terminal region of Grbl4 is not only required, but is also sufficient, for binding 2.2412. This supports the hypothesis that 2.2412 represents a general effector for the Grb7 family, since the N-terminal region of these proteins contains a highly conserved proline-rich motif which may mediate this interaction.

## Claims

1. An isolated polynucleotide molecule encoding a candidate effector protein for the Grb7 family of signalling proteins, wherein the polynucleotide molecule comprises a nucleotide sequence having at least 75% sequence identity to that shown as SEQ ID NO: 1.

2. A polynucleotide molecule according to claim 1, wherein the polynucleotide molecule comprises a nucleotide sequence having at least 85% sequence identity to that shown as SEQ ID NO: 1.

3. A polynucleotide molecule according to claim 1, wherein the polynucleotide molecule comprises a nucleotide sequence having at least 95% sequence identity to that shown as SEQ ID NO: 1.

4. A polynucleotide molecule according to claim 1, wherein the polynucleotide molecule comprises the nucleotide sequence set forth in SEQ ID NO: 1.

5. A host cell transformed with a polynucleotide molecule encoding a candidate effector protein for the Grb7 family of signalling proteins according to any one of the preceding claims.

6. A host cell according to claim 5, wherein the host cell is a mammalian, insect, yeast or bacterial host cell.

7. A method of producing a protein, comprising culturing the host cell of claim 5 or 6 under conditions suitable for the expression of the polynucleotide molecule and optionally recovering the protein.

8. A purified protein encoded by a polynucleotide molecule according to any one of claims 1 to 4.

9. A purified protein according to claim 8, wherein the protein comprises an amino acid sequence shown as SEQ ID NO: 2.

10. A fusion protein comprising a candidate effector protein for the Grb7 family of signalling proteins wherein said candidate effector protein comprises an amino acid sequence encoded by the polynucleotide molecule according to any one of claims 1 to 4.

11. A fusion protein according to claim 10 comprising the amino acid sequence shown as SEQ ID NO: 2.

12. A fusion protein according to claim 10 or claim 11, wherein said fusion protein binds to Grb14 and Grb7 proteins

13. An antibody or fragment thereof which specifically binds to a protein according to claim 8 or 9.

14. A method of detecting in a sample the presence of a polypeptide encoded by the isolated polynucleotide according to any one of claims 1 to 4, the method comprising reacting the sample with an antibody or fragment thereof according to claim 13.

15. The isolated polynucleotide according to any one of claims 1 to 4 wherein the encoded candidate effector protein for the Grb7 family of signalling proteins binds to Grb14 and Grb 7 proteins.

16. The purified protein according to claim 8 or 9 wherein said protein binds to Grb14 and Grb 7 proteins.

## Patentansprüche

1. Isoliertes Polynukleotidmolekül, welches einen Kandidaten für ein Effektorprotein für die Grb7-Familie von signalübermittelnden Proteinen codiert, wobei das Polynukleotidmolekül eine Nukleotidsequenz mit wenigstens 75% Sequenzidentität zu derjenigen, die als SEQ ID NO:1 gezeigt ist, aufweist.

2. Polynukleotidmolekül nach Anspruch 1, wobei das Polynukleotidmolekül eine Nukleotidsequenz mit wenigstens 85% Sequenzidentität zu derjenigen, die als SEQ ID NO:1 gezeigt ist, aufweist.

3. Polynukleotidmolekül nach Anspruch 1, wobei das Polynukleotidmolekül eine Nukleotidsequenz mit wenigstens 95% Sequenzidentität zu derjenigen, die als SEQ ID NO:1 gezeigt ist, aufweist.

4. Polynukleotidmolekül nach Anspruch 1, wobei das Polynukleotidmolekül die in SEQ ID NO:1 angegebene Nukleotidsequenz aufweist.

5. Wirtszelle, die mit einem Polynukleotidmolekül transformiert ist, welches einen Kandidaten für ein Effektorprotein für die Grb7-Familie von signalübermittelnden Proteinen codiert, nach einem der vorangegangenen Ansprüche.

6. Wirtszelle nach Anspruch 5, wobei die Wirtszelle eine Säuger-, Insekten-, Hefe- oder Bakterienwirtszelle ist.

7. Verfahren zur Herstellung eines Proteins, welches umfaßt, daß man die Wirtszelle nach Anspruch 5 oder 6 unter Bedingungen, die für die Expression des Polynukleotidmoleküls geeignet sind, kultiviert und optional das Protein gewinnt.

8. Gereinigtes Protein, welches von einem Polynukleotidmolekül nach einem der Ansprüche 1 bis 4 codiert wird.

9. Gereinigtes Protein nach Anspruch 8, wobei das Protein eine Aminosäuresequenz aufweist, die als SEQ ID NO:2 gezeigt ist.

10. Fusionsprotein, umfassend einen Kandidaten für ein Effektorprotein für die Grb7-Familie von signalübermittelnden Proteinen, wobei der Kandidat für ein Effektorprotein eine Aminosäuresequenz aufweist, die von dem Polynukleotidmolekül nach einem der Ansprüche 1 bis 4 codiert wird.

11. Fusionsprotein nach Anspruch 10, welches die Aminosäuresequenz aufweist, die als SEQ ID NO:2 gezeigt ist.

12. Fusionsprotein nach Anspruch 10 oder Anspruch 11, wobei das Fusionsprotein an Grb14-und Grb7-Proteine bindet.

13. Antikörper oder Fragment davon, welcher/welches spezifisch an ein Protein nach Anspruch 8 oder 9 bindet.

14. Verfahren zum Feststellen des Vorhandenseins eines Polypeptids, das von dem isolierten Polynukleotid nach einem der Ansprüche 1 bis 4 codiert wird, in einer Probe, wobei das Verfahren umfaßt, daß man die Probe mit einem Antikörper oder Fragment davon nach Anspruch 13 umsetzt.

15. Isoliertes Polynukleotid nach einem der Ansprüche 1 bis 4, wobei der codierte Kandidat für ein Effektorprotein für die Grb7-Familie von signalübermittelnden Proteinen an Grb14- und Grb7-Proteine bindet.

16. Gereinigtes Protein nach Anspruch 8 oder 9, wobei das Protein an Grb14- und Grb7-Proteine bindet.

## Revendications

1. Molécule polynucléotidique isolée codant pour une protéine effectrice candidate pour la famille Grb7 des protéines de transmission de signal, ladite molécule polynucléotidique comprenant une séquence de nucléotides présentant une identité de séquence d'au moins 75 % avec celle représentée par la SEQ ID N°1.

2. Molécule polynucléotidique suivant la revendication 1, ladite molécule polynucléotidique comprenant une séquence de nucléotides présentant une identité de séquence d'au moins 85 % avec celle représentée par la SEQ ID N°1.

3. Molécule polynucléotidique suivant la revendication 1, ladite molécule polynucléotidique comprenant une séquence de nucléotides présentant une identité de séquence d'au moins 95 % avec celle représentée par la SEQ ID N°1.

4. Molécule polynucléotidique suivant la revendication 1, ladite molécule polynucléotidique comprenant la séquence de nucléotides représentée dans la SEQ ID N°1.

5. Cellule hôte transformée avec une molécule polynucléotidique codant pour une protéine effectrice candidate pour la famille Grb7 des protéines de transmission de signal suivant l'une quelconque des revendications précédentes.

6. Cellule hôte suivant la revendication 5, ladite cellule hôte étant une cellule hôte de mammifère, d'insecte, de levure ou de bactérie.

7. Procédé pour la production d'une protéine, comprenant les étapes consistant à cultiver la cellule hôte suivant la revendication 5 ou 6 dans des conditions convenables pour l'expression de la molécule polynucléotidique et, éventuellement, à recueillir la protéine.

8. Protéine purifiée codée par une molécule polynucléotidique suivant l'une quelconque des revendications 1 à 4.

9. Protéine purifiée suivant la revendication 8, ladite protéine comprenant une séquence d'aminoacides représentée par la SEQ ID N°2.

10. Protéine de fusion comprenant une protéine effectrice candidate pour la famille Grb7 de protéines de transmission de signal, dans laquelle ladite protéine effectrice candidate comprend une séquence d'aminoacides codée par la molécule polynucléotidique suivant l'une quelconque des revendications 1 à 4.

11. Protéine de fusion suivant la revendication 10, comprenant la séquence d'aminoacides représentée par la SEQ ID N°2.

12. Protéine de fusion suivant la revendication 10 ou la revendication 11, ladite protéine de fusion se liant aux protéines Grb14 et Grb7.

13. Anticorps ou son fragment qui se lie spécifiquement à une protéine suivant la revendication 8 ou 9.

14. Méthode pour détecter dans un échantillon la présence d'un polypeptide codé par le polynucléotide isolé suivant l'une quelconque des revendications 1 à 4, méthode comprenant la réaction de l'échantillon avec un anticorps ou son fragment suivant la revendication 13.

15. Polynucléotide isolé suivant l'une quelconque des revendications 1 à 4, dans lequel la protéine effectrice candidate codée pour la famille Grb7 de protéines de transmission de signal se lie au protéines Grb14 et Grb7.

16. Protéine purifiée suivant la revendication 8 ou 9, ladite protéine se liant aux protéines Grb14 et Grb7.
